(19)

Europäisches
Patentamt
European
Patent Office
Office européen
des brevets

(11) **EP 3 451 212 A1**

(12) **EUROPEAN PATENT APPLICATION**

(43) Date of publication:
**06.03.2019  Bulletin 2019/10**

(51) Int Cl.:
***G06F 19/00*** *(2018.01)*

(21) Application number: **18172033.5**

(22) Date of filing: **14.05.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **05.09.2017   JP 2017170505**

(71) Applicant: **Sumitomo Heavy Industries, Ltd. Tokyo 141-6025 (JP)**

(72) Inventor: **MATSUMIYA, Nariaki
Yokosuka-shi,
Kanagawa 237-8555 (JP)**

(74) Representative: **Louis Pöhlau Lohrentz
Patentanwälte
Postfach 30 55
90014 Nürnberg (DE)**

(54) **SIMULATION METHOD AND SIMULATION APPARATUS FOR SIMULATING THE BEHAVIOR OF THE PLURALITY OF BEADS**

(57)    Provided is a simulation method capable of analyzing a time-variant physical quantity by using a PIMD method. In the present simulation method, a plurality of beads corresponding to each particle of a system including a plurality of particles are defined, and a path-integral molecular dynamics method of analyzing behaviors of the plurality of beads is used. The masses of the plurality of beads are set such that the masses of the plurality of beads corresponding to a single particle are the same as each other, and a sum of the masses of the plurality of beads corresponding to the single particle is the same as the mass of the corresponding particle. Behaviors of the plurality of beads corresponding to the plurality of particles are simulated by using a molecular dynamics method.

FIG. 1

```
              START
                │
   SET INITIAL CONDITIONS FOR SIMULATION      ─── S1
   TARGET SYSTEM INCLUDING PLURAL PARTICLES
                │
   DEFINE PLURAL BEADS CORRESPONDING TO       ─── S2
   EACH PARTICLE
                │
   SET MASS OF EACH OF PLURAL BEADS           ─── S3
                │
   SET INITIAL CONDITIONS FOR EACH OF PLURAL  ─── S4
   BEADS
                │
   SIMULATE BEHAVIORS OF PLURAL BEADS BY      ─── S5
   USING MOLECULAR DYNAMICS METHOD
                │
   OUTPUT SIMULATION RESULT                   ─── S6
                │
               END
```

EP 3 451 212 A1

**Description**

BACKGROUND OF THE INVENTION

Field of the Invention

Certain embodiments of the present invention relate to a simulation method and a simulation apparatus using a molecular dynamics method.

Priority is claimed to Japanese Patent Application No. 2017-170505, filed September 5, 2017, the entire content of which is incorporated herein by reference.

Description of Related Art

There is a path-integral molecular dynamics method (PIMD method) in which a system based on quantum mechanics is expressed according to classical mechanics and is analyzed. In the PIMD method, a single quantum particle is expressed by P beads in a classical system. Such a bead is referred to as a replica in some cases. The P beads are annularly connected to each other via, for example, springs. In addition to spring force, force caused by the same type of potential as a potential acting on the original particle acts on a plurality of beads. A statistical average of physical quantities is obtained by analyzing behaviors of a plurality of beads by using the molecular dynamics method under this condition. The classical statistical average obtained according to the PIMD method corresponds to a statistical average in the original quantum system (David Chandlerand Peter G. Wolynes, "Exploiting the isomorphism between quantum theory and classical statistical mechanics of polyatomic fluids", The Journal of Chemical Physics 74.7, 4078-4095 (1981)).

Serial numbers assigned to a plurality of beads representing each of original particles are related to imaginary time evolution of a quantum system, and a physical meaning of time evolution in the PIMD method is not clear. Thus, in the PIMD method of the related art, a time-independent physical quantity (a static or steady amount; a statistical average) can be computed, but a dynamic physical quantity cannot be computed.

SUMMARY OF THE INVENTION

An object of the present invention is to provide a simulation method and a simulation apparatus capable of analyzing a time-variant physical quantity by using a PIMD method.

According to an aspect of the present invention, there is provided a simulation method of defining a plurality of beads corresponding to each particle of a system including a plurality of particle, and using a path-integral molecular dynamics method of analyzing behaviors of the plurality of beads, the simulation method including setting the masses of the plurality of beads such that the masses of the plurality of beads corresponding to a single particle are the same as each other, and a sum of the masses of the plurality of beads is the same as the mass of the corresponding particle; and simulating behaviors of the plurality of beads corresponding to the plurality of particles by using a molecular dynamics method.

According to another aspect of the present invention, there is provided a simulation apparatus including an input/output device and a processing device, in which the processing device defines a plurality of beads corresponding to each particle of a system including a plurality of particle on the basis of simulation conditions which are input from the input/output device, sets the masses of the plurality of beads such that the masses of the plurality of beads corresponding to a single particle are the same as each other, and a sum of the masses of the plurality of beads is the same as the mass of the corresponding particle, and simulates behaviors of the plurality of beads corresponding to the plurality of particles by using a molecular dynamics method, and outputs a simulation result to the input/output device.

It is possible to analyze a time-variant physical quantity by using a PIMD method by setting the masses of a plurality of beads such that a sum of the masses of the plurality of beads corresponding to a single particle is the same as the mass of the corresponding particle.

BRIEF DESCRIPTION OF THE DRAWINGS

Fig. 1 is a flowchart illustrating a procedure of a simulation method according to the present embodiment.
Fig. 2 is a diagram illustrating that a plurality of (P) beads are defined for each of a plurality of particles.
Figs. 3A, 3B, and 3C are graphs respectively illustrating a first simulation result, a second simulation result, and a third simulation result.

Fig. 4 is a block diagram illustrating a simulation apparatus according to an embodiment.

DETAILED DESCRIPTION OF THE INVENTION

First, prior to description of embodiments of the present invention, a description will be made of a relationship between a distribution function $Z_{QM}$ of the quantum system handled in quantum mechanics and a classical distribution function Zp handled in classical mechanics.

The distribution function $Z_{QM}$ of the quantum system is expressed by the following equation.

$$Z_{QM} = Tr\left( e^{-\beta \hat{H}} \right) \cdots (1)$$

$$\beta = \frac{1}{k_B T}$$

Here, the H-hat is Hamiltonian, T is a temperature, and $k_B$ is a Boltzmann constant. The symbol Tr indicates a diagonal partial sum of a matrix.

A single quantum particle is expressed by P beads (replicas) in the classical system. A coordinate of an s-th bead is indicated by r(s) (where s=1, 2, 3, ..., and P). For convenience, it is assumed that $r^{(P+1)}=r^{(1)}$. The classical distribution function Zp is expressed by the following equation in a single particle system at a potential V(r).

$$Z_P = \left( \frac{mP}{2\pi\hbar^2\beta} \right)^{\frac{3P}{2}} \int \left( \prod_{s=1}^{P} d^3 r^{(s)} \right) \exp(-\beta V_{eff}) \cdots (2)$$

$$V_{eff} = \frac{1}{P} \sum_{s=1}^{P} V\left( r^{(s)} \right) + \frac{1}{2} m\omega_P^2 \sum_{s=1}^{P} \left( r^{(s)} - r^{(s+1)} \right)^2$$

$$\omega_P = \frac{\sqrt{P}}{\hbar\beta}$$

Here, P is an integer, and m indicates the mass of a quantum particle. P beads corresponding to a single particle are annularly connected to each other via springs as indicated by the second term of the right side of the equation representing an effective potential $V_{eff}$.

The distribution function $Z_{QM}$ of the quantum system shown in Equation (1) is the same as a result of the parameter P taking the infinite limit in the classical distribution function $Z_P$ shown in Equation (2). In other words, the following relationship is established.

$$Z_{QM} = \lim_{P \to \infty} Z_P \cdots (3)$$

In the PIMD method, the parameter P of the classical distribution function $Z_P$ is set to a finite value, and a classical motion equation is solved, so that a statistical average of physical quantities is obtained. The statistical average obtained according to the classical method corresponds to a statistical average in the original quantum system. However, a physical meaning of time evolution in the PIMD method is not clear.

In the present embodiment described below, time evolution in the PIMD method is assumed to correspond to time evolution in real time.

Only the potential of the system is explicitly shown on the right side of Equation (2). Virtual momentum p(s) of the s-th bead and the mass M of each bead are introduced, and Equation (2) may be rewritten as follows.

$$Z_P = \left(\frac{mP}{4\pi^2\hbar^2 M}\right)^{\frac{3P}{2}} \int \left(\prod_{s=1}^{P} d^3 r^{(s)} d^3 p^{(s)}\right) \exp\left(-\beta H_{eff}\right) \cdots (4)$$

Here, $H_{eff}$ is expressed by the following Equation (5).

$$H_{eff} = \frac{1}{2M}\sum_{s=1}^{P}\left(p^{(s)}\right)^2$$

$$+\frac{1}{P}\sum_{s=1}^{P}V\left(r^{(s)}\right) + \frac{1}{2}m\omega_P^2\sum_{s=1}^{P}\left(r^{(s)} - r^{(s+1)}\right)^2 \cdots (5)$$

$H_{eff}$ expressed by Equation (5) may be regarded as Hamiltonian of the classical system corresponding to the original quantum system. The first term of the right side of Equation (5) indicates kinetic energy of a bead, the second term indicates potential energy replaced such that potential energy acting between original particles acts between beads, and the third term indicates potential energy caused by spring force (attractive force) acting between P beads corresponding to a single particle. As mentioned above, an interaction potential in which a potential (the second term of the right side of Equation (5)) obtained on the basis of the potential V(r) acting between the original particles is superimposed on a potential (the third term of the right side of Equation (5)) causing attraction between the beads acts between the P beads corresponding to a single particle. Hereinafter, $H_{eff}$ in Equation (5) is assumed to be Hamiltonian of the classical system, and the quantum system is compared with the classical system.

If a parameter corresponding to time evolution is indicated by $\tau$, a motion equation used in the PIMD method may be expressed as follows.

$$M\frac{d^2}{d\tau^2}r^{(s)} = -\frac{\partial}{\partial r^{(s)}}H_{eff} \cdots (6)$$

The following amount $<A>_{PIMD}$ which can be computed in the PIMD method is defined as an expected value of an operator hat-A, so as to correspond to the operator hat-A which does not depend on time in quantum mechanics and depends on only a position of a particle.

$$\langle A \rangle_{PIMD} = \frac{1}{P} \sum_{s=1}^{P} A\left(r^{(s)}\right) \cdots (7)$$

If the operator hat-A is defined as a position of a particle, the following equation is derived from Equations (6) and (7).

$$MP\frac{d^2}{d\tau^2}\langle r \rangle_{PIMD} = -\left\langle \frac{\partial}{\partial r}V(r) \right\rangle_{PIMD} \cdots (8)$$

Equation (8) is a motion equation followed by the centroid of the P beads corresponding to the original particle.

In the quantum mechanics, an expected value of the operator hat-A which does not depend on time under a wave function $\phi$ is defined by the following equation.

$$\left\langle \hat{A} \right\rangle_{QM} = \int d^3r\, \psi^*(r,t)A(r)\psi(r,t) \cdots (9)$$

An equation followed by an expected value of a position of a particle is expressed by the following equation on the basis of Equation (9) and a Schroedinger equation (Ehrenfest's theorem).

$$m\frac{d^2}{dt^2}\langle r \rangle_{QM} = -\left\langle \frac{\partial}{\partial r}V(r) \right\rangle_{QM} \cdots (10)$$

In a case where the motion equation (8) followed by the centroid of the P beads is compared with Equation (10) followed by an expected value of a position of a particle in the quantum system, it can be seen that both of the equations may have the same form at M=m/P. Therefore, if an initial condition of each particle at a time point t=0 in the quantum system is given, and a corresponding initial condition of beads at a time point $\tau$=0 in the PIMD method is given, an expected value of a position of a particle in the quantum system and a value of a position of the centroid of beads in the PIMD method are the same as each other when P is set to the infinite limit. In other words, this can be interpreted as the time $\tau$ in the PIMD method having the same physical meaning as the real time t in the quantum system.

As mentioned above, the mass M of a bead in the PIMD method is set to satisfy M=m/P, a behavior of the bead is simulated, and thus dynamic property of a particle in the quantum system can be examined. The equality M=m/P indicates that the masses of a plurality of beads corresponding to a single particle are the same as each other, and the mass of each of the plurality of beads is set such that a sum of the masses of the plurality of beads corresponding to the single particle is the same as the mass of the corresponding particle.

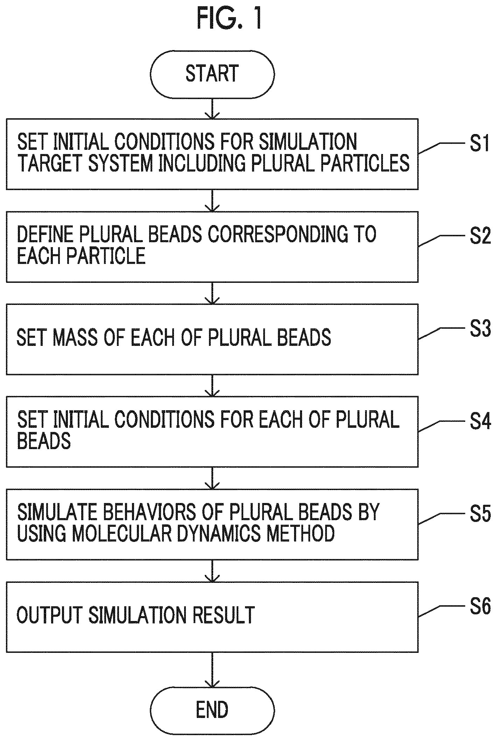

Fig. 1 is a flowchart illustrating a procedure of a simulation method according to the present embodiment. Each of the following processes is performed by a simulation apparatus. First, initial conditions for a simulation target system including a plurality of particles are set (step S1). The initial conditions include a position and a velocity of a particle. Interaction potentials acting between particles, boundary conditions, and the like are set. Basis information for setting the initial conditions, the interaction potentials, the boundary conditions, and the like is given by a user inputting the basis information to the simulation apparatus.

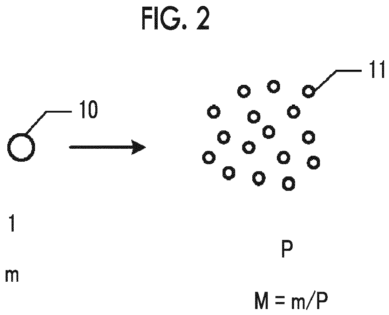

As illustrated in Fig. 2, the simulation apparatus defines a plurality of (P) beads 11 for each of a plurality of particles 10 (step S2). The number P of beads corresponding to a single particle is an integer of 2 or greater. The mass M of each of the plurality of beads is set (step S3). In the embodiment, the mass M is set to satisfy M=m/P such that the time $\tau$ in the PIMD method has the same physical meaning as that of the real time t in the quantum system. Here, m indicates the mass of an original particle corresponding to P beads.

Thereafter, the simulation apparatus sets initial conditions for each of the plurality of beads on the basis of the initial conditions for the original particle (step S4). For example, initial conditions for each bead are set such that a centroid position of the P beads matches a position of the original particle, and an average velocity of the P beads matches a velocity of the original particle.

Next, behaviors of the plurality of beads are simulated by using a molecular dynamics method (step S5). In this case, the motion Equation of Equation (6) is used. If the simulation is completed, a simulation result is output (step S6). For example, a temporal change in a position of the centroid of the P beads corresponding to each particle is displayed as a graphic.

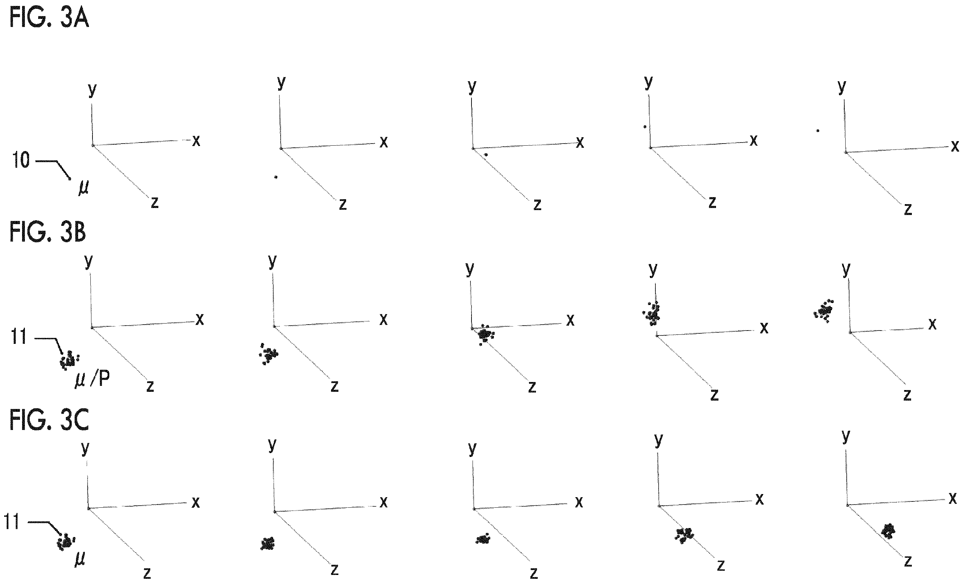

Next, with reference to Figs. 3A to 3C, a description will be made of excellent effects of the embodiment by exemplifying simulation in which an electron is caused to collide with a proton.

First, simulation conditions will be described. The motions of a proton with a coordinate $r_p$ and the mass $m_p$ and an electron with a coordinate $r_e$ and the mass $m_e$ are converted into a centroid coordinate R and a relative coordinate r. The centroid coordinate R and the relative coordinate r are expressed by the following equations.

$$R = \frac{m_p r_p + m_e r_e}{m_p + m_e}$$

$$r = r_e - r_p \cdots (11)$$

The centroids of the proton and the electron are subjected to linear uniform motion. The relative coordinate r follows a motion equation of a particle with conversion mass $\mu$ at the potential V(r). The potential V(r) and the conversion mass $\mu$ are expressed by the following equations.

$$V(r) = -\frac{e^2}{4\pi\epsilon_0 r}$$

$$\mu = \frac{m_p m_e}{m_p + m_e} \cdots (12)$$

Here, e indicates elementary electric charge, and $\epsilon_0$ indicates a dielectric constant of vacuum.

In this simulation, a temporal change in the relative coordinate r was obtained. However, since the potential V(r) and force acting on a particle diverge at r=0, a rounded potential tilde-A was used as follows in the simulation instead of the potential V(r) in Equation (12) by using an error function erf and the Bohr radius ao such that the potential and the force do not diverge near r=0.

$$\tilde{V}(r) = -\frac{e^2}{4\pi\epsilon_0 r} erf\left(\frac{10r}{a_0}\right) \cdots (13)$$

The following three types of simulations were performed. In the first simulation, a temporal change in a position of a classical particle with the conversion mass $\mu$ was obtained according to the molecular dynamics method. A position of the classical particle is indicated by $r_1$, and a velocity thereof is indicated by $v_1$.

In the second simulation, the quantum system was described according to the PIMD method, and temporal changes in positions of the P beads were obtained. P was set to 32, and the mass of each of the beads was set to μ/P. A position of a bead is indicated by $r_2^{(s)}$, and a velocity thereof is indicated by $v_2^{(s)}$. Here, s is a serial number assigned to a bead, and is an integer of 1 or greater and P or smaller.

In the third simulation, the mass of each of beads was set to μ. Other conditions are the same as the conditions in the second simulation.

An initial value of a position of the classical particle in the first simulation, an initial value of a position of the centroid of the P beads in the second simulation, and an initial value of a position of the centroid of the P beads in the third simulation were caused to be the same as each other. An initial value of a velocity of the classical particle in the first simulation, an initial value of an average velocity of the P beads in the second simulation, and an initial value of an average velocity of the P beads in the third simulation were caused to be the same as each other. Initial values of positions of the P beads in the second simulation and initial values of positions of the P beads in the third simulation were caused to be the same as each other. An initial value of a velocity of each of the P beads in the second simulation and an initial value of a velocity of each of the P beads in the third simulation were caused to be the same as each other.

In other words, the initial conditions satisfy the following equations.

$$r_1(t=0) = \frac{1}{P}\sum_{s=1}^{P} r_2^{(s)}(t=0) = \frac{1}{P}\sum_{s=1}^{P} r_3^{(s)}(t=0)$$

$$v_1(t=0) = \frac{1}{P}\sum_{s=1}^{P} v_2^{(s)}(t=0) = \frac{1}{P}\sum_{s=1}^{P} v_3^{(s)}(t=0)$$

$$r_2^{(s)}(t=0) = r_3^{(s)}(t=0)$$

$$v_2^{(s)}(t=0) = v_3^{(s)}(t=0) \cdots (14)$$

Figs. 3A, 3B, and 3C are graphs respectively illustrating a first simulation result, a second simulation result, and a third simulation result. In Figs. 3A, 3B, and 3C, the centroid coordinate R was fixed to the origin. The centroid coordinate R substantially corresponds to a position of a proton. The graphs of Figs. 3A, 3B, and 3C indicate temporal changes in the relative coordinate r at the potential tilde-V. The relative coordinate r substantially indicates a position of an electron. In each of Figs. 3A, 3B, and 3C, five graphs arranged horizontally indicate that time elapses from the left graph toward the right graph.

A locus of the centroid of the P beads 11 illustrated in Fig. 3B substantially matches a locus of the particle 10 illustrated in Fig. 3A, obtained through classical analysis. However, the centroid of the P beads 11 illustrated in Fig. 3C shows a behavior which is completely different from a behavior of the particle 10 illustrated in Fig. 3A, obtained through classical analysis. From this, it can be seen that a result of setting the mass of each of a plurality of beads corresponding to a particle of the quantum system to 1/P times the mass of an original particle and performing simulation by using the PIMD method provides accurate time evolution corresponding to the classical system. In contrast, in a case where the mass of each of a plurality of beads corresponding to a particle of the quantum system is set to other values, and simulation is performed according to the PIMD method, it cannot be said that time evolution in the PIMD method corresponds to time evolution in the original particle system.

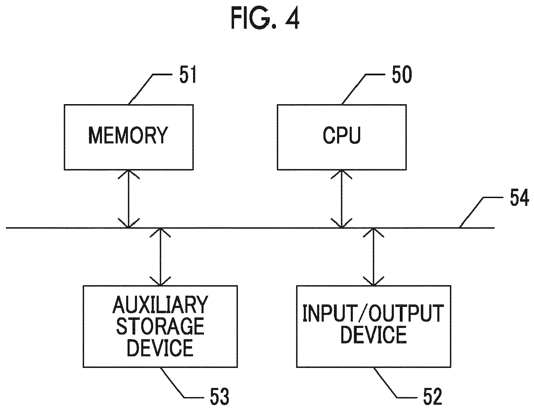

Next, with reference to Fig. 4, a description will be made of the simulation apparatus according to the embodiment. Fig. 4 is a block diagram illustrating the simulation apparatus according to the embodiment. The simulation apparatus is formed of a general computer, and includes a central processing unit (CPU; a processing device) 50, a memory 51, an input/output device 52, and an auxiliary storage device 53. The constituent elements are connected to each other via a bus 54.

The input/output device 52 includes pointing devices such as a keyboard and a mouse, a display, a reader/writer for removable media, a communication device, and the like. The display displays various windows required for a user to perform an operation, data, or the like. The communication device performs data communication with an external apparatus. The user performs the input/output device so as to give an instruction for the computer and to input data required in each process. A process result is output to the input/output device.

The auxiliary storage device 53 is formed of a hard disk or the like, and stores an OS of the computer, a simulation program, data or a process result required in simulation, and the like.

The memory 51 is formed of a read only memory (ROM), a random access memory (RAM), or the like. The memory 51 stores the simulation program or the like read from the auxiliary storage device 53 by the CPU 50.

The CPU 50 performs various calculations or control of the input/output device 52 and the auxiliary storage device 53 on the basis of the OS of the computer, the simulation program stored in the memory 51, and the like.

Next, a description will be made of an operation of the simulation apparatus. The CPU 50 acquires simulation conditions such as initial conditions from the input/output device 52 in order to perform the process in step S1 (Fig. 1). For example, the user operates the input/output device 52 so as to input the simulation conditions such as initial conditions. Alternatively, the CPU 50 reads the simulation conditions such as initial conditions from a removable medium. Thereafter, the CPU 50 performs the processes in steps S2 to S5. In step S6, the CPU 50 displays, for example, an analysis result on the display as graphics.

The embodiment is only an example, and an embodiment of the present invention is not limited to the embodiment. For example, it is clear to a person skilled in the art that vibrator alterations, modifications, combinations, and the like may occur.

It should be understood that the invention is not limited to the above-described embodiment, but may be modified into various forms on the basis of the spirit of the invention. Additionally, the modifications are included in the scope of the invention.

Brief Description of the Reference Symbols

10: particle
11: bead
50: central processing unit (CPU; processing device)
51: memory
52: input/output device
53: auxiliary storage device
54: bus

**Claims**

1. A simulation method of defining a plurality of beads corresponding to each particle of a system including a plurality of particles, and using a path-integral molecular dynamics method of analyzing behaviors of the plurality of beads, the simulation method comprising:

   setting the masses of the plurality of beads such that the masses of the plurality of beads corresponding to a single particle are the same as each other, and a sum of the masses of the plurality of beads corresponding to the single particle is the same as the mass of the corresponding particle; and
   simulating behaviors of the plurality of beads corresponding to the plurality of particles by using a molecular dynamics method.

2. The simulation method according to claim 1,
   wherein, when simulation is performed by using the molecular dynamics method, a potential in which a potential obtained on the basis of a potential acting between the particles is superimposed on a potential causing attraction between the beads is introduced among the plurality of beads corresponding to the single particle.

3. A simulation apparatus comprising:

   an input/output device and a processing device,

wherein the processing device

defines a plurality of beads corresponding to each particle of a system including a plurality of particles on the basis of simulation conditions which are input from the input/output device,

sets the masses of the plurality of beads such that the masses of the plurality of beads corresponding to a single particle are the same as each other, and a sum of the masses of the plurality of beads corresponding to the single particle is the same as the mass of the corresponding particle, and

simulates behaviors of the plurality of beads corresponding to the plurality of particles by using a molecular dynamics method, and outputs a simulation result to the input/output device.

4. The simulation apparatus according to claim 3,
   wherein the processing device defines the plurality of beads such that a centroid position and an average velocity of the plurality of beads corresponding to each of the particles match initial conditions for a position and a velocity of the corresponding particle.

5. The simulation apparatus according to claim 3 or 4,
   wherein, when simulation is performed by using the molecular dynamics method, the processing device introduces a potential in which a potential obtained on the basis of a potential acting between the particles is superimposed on a potential causing attraction between the beads among the plurality of beads corresponding to the single particle.

6. The simulation apparatus according to any one of claims 3 to 5,
   wherein the simulation result which is output to the input/output device includes a temporal change in a centroid position of the plurality of beads corresponding to each of the plurality of particles.

# FIG. 1

START

SET INITIAL CONDITIONS FOR SIMULATION TARGET SYSTEM INCLUDING PLURAL PARTICLES — S1

DEFINE PLURAL BEADS CORRESPONDING TO EACH PARTICLE — S2

SET MASS OF EACH OF PLURAL BEADS — S3

SET INITIAL CONDITIONS FOR EACH OF PLURAL BEADS — S4

SIMULATE BEHAVIORS OF PLURAL BEADS BY USING MOLECULAR DYNAMICS METHOD — S5

OUTPUT SIMULATION RESULT — S6

END

FIG. 2

1

m

M = m/P

FIG. 3A

FIG. 3B

FIG. 3C

FIG. 4

```
        ┌──── 51                    ┌──── 50
   ┌──────────────┐           ┌──────────────┐
   │    MEMORY    │           │     CPU      │
   └──────────────┘           └──────────────┘
          ↕                          ↕
                                                    ┌──── 54
   ─────────────────────────────────────────────
          ↕                          ↕
   ┌──────────────┐           ┌──────────────┐
   │  AUXILIARY   │           │ INPUT/OUTPUT │
   │   STORAGE    │           │    DEVICE    │
   │    DEVICE    │           └──────────────┘
   └──────────────┘               └──── 52
        └──── 53
```

**EP 3 451 212 A1**

# EUROPEAN SEARCH REPORT

Application Number

EP 18 17 2033

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PINILLA C. ET AL: "Equilibrium fractionation of H and O isotopes in water from path integral molecular dynamics", GEOCHIMICA ET COSMOCHIMICA ACTA, vol. 135, 29 March 2014 (2014-03-29), pages 203-216, XP028648589, ISSN: 0016-7037, DOI: 10.1016/J.GCA.2014.03.027 \* abstract \* \* page 205, right-hand column, line 14 - page 207, right-hand column, line 13 \* \* figure 1 \* | 1-6 | INV. G06F19/00 |
| X | AGARWAL A. ET AL: "Grand-Canonical Adaptive Resolution Centroid Molecular Dynamics: Implementation and application", COMPUTER PHYSICS COMMUNICATION, ELSEVIER SCIENCE PUBLISHERS B.V., AMSTERDAM, NL, vol. 206, 14 May 2016 (2016-05-14), pages 26-34, XP029595825, ISSN: 0010-4655, DOI: 10.1016/J.CPC.2016.05.001 \* paragraph [1.1.] \* \* figure 1 \* | 1-6 | |
| | -/-- | | **TECHNICAL FIELDS SEARCHED (IPC)**<br><br>G06F |

The present search report has been drawn up for all claims

| Place of search | Date of completion of the search | Examiner |
|---|---|---|
| The Hague | 5 October 2018 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

**EUROPEAN SEARCH REPORT**

Application Number

EP 18 17 2033

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DOPIERALSKI P. D. ET AL: "Proton distribution in KHCO"3 from abinitio molecular dynamics simulation", CHEMICAL PHYSICS LETTERS, ELSEVIER BV, NL, vol. 476, no. 4-6, 16 July 2009 (2009-07-16), pages 223-226, XP026282440, ISSN: 0009-2614, DOI: 10.1016/J.CPLETT.2009.06.048 [retrieved on 2009-06-21] * abstract * * page 223, right-hand column, line 12 - page 224, left-hand column, line 20 * * figures 2,3 * | 1-6 | |
| X | IVANOV S. D. ET AL: "Bead-Fourier path integral molecular dynamics", PHYSICAL REVIEW E. STATISTICAL PHYSICS, PLASMAS, FLUIDS, AND RELATED INTERDISCIPLINARY TOPICS., vol. 67, no. 6, 1 June 2003 (2003-06-01), page 066710, XP055512151, US ISSN: 1063-651X, DOI: 10.1103/PhysRevE.67.066710 * the whole document * | 1-6 | |
| A | US 2014/229150 A1 (TSUMURA KYOSUKE [JP] ET AL) 14 August 2014 (2014-08-14) * paragraph [0069] - paragraph [0074] * | 1-6 | TECHNICAL FIELDS SEARCHED (IPC) |
| A | EP 2 793 150 A1 (SUMITOMO RUBBER IND [JP]) 22 October 2014 (2014-10-22) * paragraph [0057] - paragraph [0059] * | 1-6 | |
| A | US 2015/254378 A1 (ARTEMOVA SVETLANA [FR] ET AL) 10 September 2015 (2015-09-10) * abstract * | 1-6 | |

The present search report has been drawn up for all claims

| Place of search | Date of completion of the search | Examiner |
|---|---|---|
| The Hague | 5 October 2018 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 17 2033

This annex lists the patent family members relating to the patent documents cited in the above-mentioned European search report.
The members are as contained in the European Patent Office EDP file on
The European Patent Office is in no way liable for these particulars which are merely given for the purpose of information.

05-10-2018

| Patent document cited in search report | | | Publication date | Patent family member(s) | | | Publication date |
|---|---|---|---|---|---|---|---|
| US 2014229150 | A1 | | 14-08-2014 | BR | 112014006614 | A2 | 25-04-2017 |
| | | | | CN | 103827875 | A | 28-05-2014 |
| | | | | EP | 2761519 | A1 | 06-08-2014 |
| | | | | JP | 5697638 | B2 | 08-04-2015 |
| | | | | JP | 2013084255 | A | 09-05-2013 |
| | | | | RU | 2014117019 | A | 10-11-2015 |
| | | | | US | 2014229150 | A1 | 14-08-2014 |
| | | | | WO | 2013047881 | A1 | 04-04-2013 |
| EP 2793150 | A1 | | 22-10-2014 | EP | 2793150 | A1 | 22-10-2014 |
| | | | | JP | 6097130 | B2 | 15-03-2017 |
| | | | | JP | 2014206913 | A | 30-10-2014 |
| | | | | KR | 20140123915 | A | 23-10-2014 |
| | | | | US | 2014309971 | A1 | 16-10-2014 |
| US 2015254378 | A1 | | 10-09-2015 | CN | 104756115 | A | 01-07-2015 |
| | | | | EP | 2893473 | A1 | 15-07-2015 |
| | | | | FR | 2995109 | A1 | 07-03-2014 |
| | | | | US | 2015254378 | A1 | 10-09-2015 |
| | | | | WO | 2014037236 | A1 | 13-03-2014 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017170505 A

**Non-patent literature cited in the description**

- **DAVID CHANDLER ; PETER G. WOLYNES.** Exploiting the isomorphism between quantum theory and classical statistical mechanics of polyatomic fluids. *The Journal of Chemical Physics,* 1981, vol. 74.7, 4078-4095